(19) Europäisches Patentamt
European Patent Office
Office européen des brevets

(11) Veröffentlichungsnummer: **0 060 994**
**A1**

(12) # EUROPÄISCHE PATENTANMELDUNG

(21) Anmeldenummer: 82101087.3

(22) Anmeldetag: 13.02.82

(51) Int. Cl.³: C 07 D 213/30
A 61 K 31/44
//C07C149/20

(30) Priorität: 17.03.81 IT 2037081

(43) Veröffentlichungstag der Anmeldung: 29.09.82 Patentblatt 82/39

(84) Benannte Vertragsstaaten: AT BE CH DE FR GB LI LU NL SE

(71) Anmelder: LABORATORI PROPHIN S.p.A.
Via Binda, 21
I-20143 Mailand(IT)

(72) Erfinder: Bin, Anna Maria
Via Beato Pellegrino 96
Padua(IT)

(74) Vertreter: Bianchetti, Giuseppe
Studio Consulenza Brevettuale Via Senato 24
I-20121 Milan(IT)

(54) **Ein neues Derivat des Bis-hydroxyethylmercapto-1,10-decans, ein Verfahren für seine Herstellung sowohl den Wirkstoff enthaltende pharmazeutische Zubereitungen.**

(57) Gegenstand der Erfindung ist ein neues Derivat des Bis-hydroxyethylmercapto-1, 10-decans der Formel I

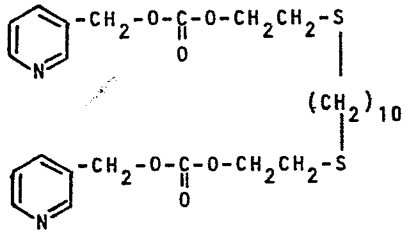

1

Die neue Verbindung weist ausgezeichnete hypolipidämische Eigenschaften auf; ein weiterer Gegenstand der Erfindung sind daher auch die neue Verbindung als Winkstoff enthaltenden pharmazeutische Zubereitungen.

EP 0 060 994 A1

Croydon Printing Company Ltd.

Ein neues Derivat des Bis-hydroxyethylmercapto-1,10-decans, ein Verfahren für seine Herstellung sowohl den Wirkstoff enthaltende pharmazeutische Zubereitungen

Die vorliegende Erfindung bezieht sich auf ein neues Derivat des Bis-hydroxyethylmercapto-1,10-decans der Formel I

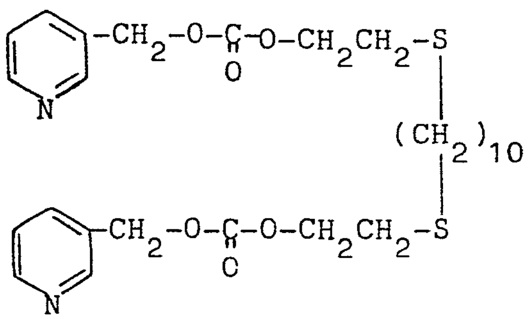

I

sowie auf dessen Salze von pharmazeutisch annehmbaren Säuren.

Die erfindungsgemässe Verbindung weist ausgezeichnete hypolipidämische Eigenschaften auf, so dass sie Anwendung in der Behandlung der Atherosklerosen von verschiedener Natur finden kann.

Daher, bezieht sich die Erfindung auch auf pharmazeutische Zubereitungen mit lypolysishemmender Wirkung, die die Verbindung der Formel I sowie deren Salze von pharmazeutisch annehmbaren Säuren als Wirkstoff enthalten.

Schliesslich ist ein weiterer Gegenstand der vorliegenden Erfindung ein Verfahren für die Herstellung der Verbindung der Formel I, dadurch gekennzeichnet, dass man in an sich bekannter Weise die Verbindung

der Formel II (bekannt aus der Literatur)

$$Cl-COO-CH_2CH_2-S-(CH_2)_{10}-S-CH_2CH_2-OCO-Cl$$

II

mit 3-Pyridylmethanol der Formel III

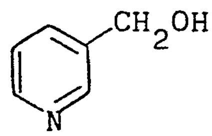

III

Alternativ kann man die erfindungsgemässe Substanz durch Kondensation von 3-Pyridylmethanolchlorcarbonat der Formel IV

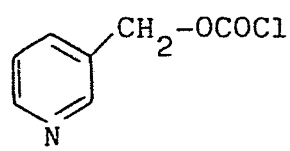

IV

mit Bis-hydroxyethylmercapto-1,10-decan der Formel V

$$HO-CH_2CH_2-S-(CH_2)_{10}-S-CH_2CH_2-OH$$

V

unter bekannten Bedingungen herstellen.

Die Erfindung kann durch die folgende Beispiele besser veranschaulicht werden.

Beispiel 1

Gemischtes Doppelcarbonat des Bis-hydroxyethylmercapto-1,10-decans und des 3-Pyridylmethanols

a) Bis-Hydroxyethylmercapto-1,10-decan bis-chlorcarbonat

7.4 Gramme von Bis-hydroxyethylmercapto-1,10-decan und 40 ml einer 20-prozentigen Toluollösung von Phosgen wurden in einer dreihalsigen Flasche, die mit einem Rührer, einem Rohr für das Einblasen des Stickstoffs und einem geöffneten Hals für die Einführung der Reagenzen ausgerüstet war, gerührt. Die Reaktionsmischung wurde bis zur kompletten Lösung bei Raumtemperatur gehalten, dann liess man einen Stickstoffsstrom zwecks Entfernung des Phosgenüberschusses 30-Minuten lang durchblasen.

b) Kondensation mit dem 3-Pyridylmethanol

Die gemäss a) erhaltene Toluollösung wurde erst durch einen Eisbaden gekühlt und dann einer Lösung von 25 ml von 3-Pyridylmethanol in 100 ml von Benzol tropfenweise zugegeben. Nach der Zugabe wurde die Reaktionsmischung bie Raumtemperatur 24 Stunden gerührt, der erhaltene Niederschlag, der aus dem 3-Pyridylmethanol-hydrochlorid bestand, abfiltriert wurde (so gewinnt man einen Teil des 3-Pyridylmethanols) und das Filtrat wurde im Vakuum eingeengt (so gewinnt man einen weiteren Teil von 3-Pyridylmethanol, das nach Reinigung wieder angewendet werden kann). Der ölige Rückstand, wurde durch einer kleinen Menge Isopropanol kristallisiert und durch Abfiltrieren rein isoliert. Der so erhaltene rohe Festsstoff (13 g), wurde aus der geringsten Menge von Isopropanol umkristallisiert.
Ausbeute am Endprodukt: 10,5 g eines Weissen, seidenartigen Festsstoffs; Schmelzpunkt: 65-66°C.
Elementaranalyse für $C_{28}H_{40}N_2O_6S_2$ (Mol.Gew.=564,5)
Ber.: C 59,52%; H 7,14%; N 4,96%
Gef.: C 59,80%; H 7,04%; N 4,96%
N.M.R.: 1,31 δ(s, verbreitet, $8CH_2$); 2,4-3,0 δ(m, $4S-CH_2$); 4,29 δ(t, $2COOCH_2R$); 5,18 δ(s, $2Ar-CH_2O$); 7,1-7,4 δ(m); 7,6-7,9 δ(m); 8,4-8,8 δ(m) (2H+2H+2H, aromatische).
I.R.: charakteristische Absorptionsband der Carbonylgruppe bei 1735 $cm^{-1}$

Beispiel 2

a) 3-Pyridylmethanol-chlorcarbonat

50 Milliliter einer 20-prozentigen Toluollösung von Phosgen wurden langsam mit 10,9 g von 3-Pyridylmethanol versetzt und die so erhaltene Reaktionsmischung wurde bei Raumtemperatur 24 Stunden gerührt.Man liess einen Stickstoffsstrom durchblasen und verwendete diese Lösung in der nachfolgenden Stufe.

b) Gemischtes Doppelcarbonat des Bis-hydroxyethylmercapto-1,10-decans und 3-Pyridylmethanols

Die gemäss a) hergestellte Lösung wurde langsam mit einer Benzollösung von 14,7 g von Bis-hydroxyethylmercapto-1,10-decan und 20 ml von Pyridin versetzt

und die so erhaltene Mischung wurde 10 Stunden gerührt. Man erhielt einen Niederschlag, der durch Abfiltrieren entfernt wurde, das Filtrat wurde auf klein Volumen eingeengt und der Rückstand mit wenigem Isopropanol aufgenommen. Der abgetrennte Festsstoff wurde durch Filtrieren gewonnen und das rohe Produkt wurde aus der geringsten Menge Isopropanol umkristallisiert. Ausbeute am Endprodukt: 20 g eines weissen, seidenartigen Feststoffs. Schmelzpunkt : 64-66°C. Auch in diesem Falle bestätigen die Elementaranalyse sowie die N.M.R. - und I.R.- Angaben die Struktur der erfindungsgemässen Verbindung. Von nun an soll die erfindungsgemässe Substanz auch mit der Abkürzung TN gezeichnet werden. Ihre pharmako-biologischen Eigenschaften werden im Folgenden veranschaulicht.

Die neue Verbindung TN,in deren Struktur die Moleküle des Thiadenols (T) und der Nicotinols vorliegen, besitzt eine sehr interessante lypolysishemmende Wirkung. Die in der untenanliegenden Tabelle hervorgebrachten Resultate wurden auf, aus dem Epydimalgewebe der Ratten isolierten und in vitro inkubierten Fettzellen durchgeführt, wobei die Befreiung von Glyzerol als Parameter der Lypolysis in Anbetracht gezogen wurde.

Lypolysis

| Verbindung | Spontane | +Epynephrin $10^{-6}$M | +Theophyllin $3x10^{-4}$M |
|---|---|---|---|
| - | - | x12,8 | x7,6 |
| TN $10^{-4}$M | -70% | -34% | -30% |
| TN $10^{-3}$M | -84% | -58% | -69% |
| T $10^{-4}$M | n.b. | n.b. | -34% |
| T $10^{-3}$M | n.b. | -33% | -47% |
| N $2x10^{-4}$M | -60% | n.b. | -37% |
| N $2x10^{-3}$M | -89% | -38% | -60% |
| T $10^{-4}$M+N$2x10^{-4}$M | n.b. | n.b. | -22% |
| T $10^{-3}$M+N$2x10^{-3}$M | n.b. | -28% | -49% |

n.b.= Nicht bedeutungsvolle Anderungen im Vergleich zu den Kontrollen.

Die spontane Lypolysis in Abwesenheit von Arzneimitteln wurde als Vergleichstermin in Anbetracht gezogen.
In der ersten Zeile wird die von der Epynephrin und Theophyllin in Vergleich mit der spontanen Lypolysis induzierte Zunahme angegeben.
Die Hemmungsprozentualen beziehen sich auf die spontane (erste Spalte), von Epynephrin induzierte (zweite Spalte) und von Theophyllin induzierte (dritte Spalte) Lypolysis. Alle die angegebenen Anderungen sind statistisch bedeutungsvoll.

Die Ergebnisse beziehen sich auf in 30 min./$_{10}{}^{6}$Zellen befreiten µMole von Glyzerol.

Resultate

a) Die spontane Mobilisierung der Lypide von den Fettzellen wird von N und, auf rund identischem Mass, von TN, aber nicht von T oder T+N gehemmt.

b) In Gegenwart von Epynephrin, d.h. unter Umständen, die erlauben, die in vitro Hypermobilisierung der Fettsäuren zu reproduzieren, die im Organismus infolge verschiedener Type von Stress und anderer, zu einer Zunahme der Blutepynephrin führender Ursachen stattfinden, ist die Verbindung TN noch in der Lage, die Lypidmobilisierung zu hemmen. Unter diesen Umständen hat sich erfindungsgemässe Verbindung offentlich viel wirksamer als T und N, sowohl alsder Assoziation T+N erwiesen. Diese Angabe ist von besonderer Bedeutung, indem gerade das adrenergische System auf die primäre Kontrolle des Stoffwechsels des Fettgewebes wirkt.

c) Eine weitere Ursache der übermässigen Befreiung der Fettsäuren von den Adipozyten kann der funktionellen Veränderung von besonderen, in der Kontrolle der Lypolysis verwickelten Intrazellenzymen zugeschrieben werden.
Ein solcher Umstand kann *in vitro* durch Anwendung

von Theophyllin reproduziert werden, d.h. einem Arzneimittel, das die Phosphordiesterase und, demzufolge, den normalen Katabolismus von dem zyklischen AMP hemmt. Auf dieser Weise, und hauptsächlich auch durch Interferieren mit anderen Kontrollsystemen, stimuliert die Theophyllin die Hydrolyse der TGL und, also, die Befreiung von FFA und Glyzerol. Übrigens ist für T eine <u>in vitro</u> lypolysishemmende Wirkung in Anwesenheit von zyklischem AMP-dibutyrat anerkannt, die durch Bewerbung mit dem endogenen, zyklischem AMP der Phosphordiesterase die Lypolysis stimuliert.

Auch unter diesen Umständen nimmt merklich die Verbindung TN die Lypidmobilisierung ab und erweist sich viel wirksamen als sei es N, sei es T, sei es T+N.

Im grossen und ganzen bestätigen diese Ergebnisse, dass die Verbindung TN eine ausgeprägte lypolysishemmende Wirkung <u>in vitro</u> besitzt, und mit Recht kann man voraussetzen, dass dies zu einer <u>in vivo</u> lypolipidämischer Aktivität entspricht. Darüber hinaus bestätigt die Tatsache, dass die lypolysishemmende Wirkung der erfindungsgemässen Substanz höher ist sowohl als die der einzelnen Moleküle, die an ihre Struktur teilnehmen, als auch als die der einfachen Assoziation beider Substanzen, die grosse Bedeutung der neuen chemischen Bindungen und Änderungen aus dem biologischen und pharmacologischen Standpunkt .

## Akute Toxizität

Die akute Toxizität wurde in der Maus (Swissstamm) durch orale Verbreichung einer Suspension der Verbindung TN in 10% Gummiarabikum ermittelt. Gemäss Literaturangaben weist T unter den gleichen experimentellen Bedingungen einen $LD_{50}$-Wert von 10g/Kg auf; die Toxizität der Verbindung TN ist rund der gleichen Grösse, d.h. nicht höher als die der einzelnen Verbindung T.

## Verträglichkeit im Menschen

Die Verabreichung zu gesunden Freiwilligen der Verbindung TN, verkörpert in Kapseln,die eine entsprechende Menge von Wirkstoff als eine 400 mg Kapsel von T enthalten hat keine Nebeneffekte veranlasst, auch wenn sie zwei- oder dreimal pro Tag während einer Monat fortgesetzt wurde.

Patentansprüche:

1. Neues Derivat des Bis-hydroxyethylmercapto-1,10-decans der Formel I

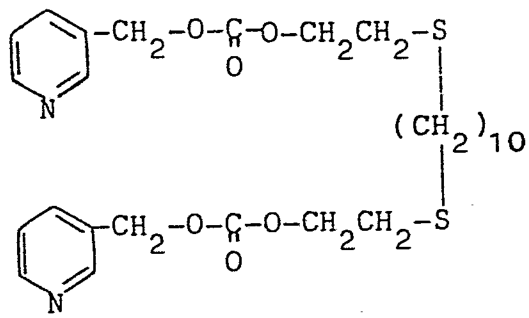

I

sowie dessen Salze von pharmazeutisch annehmbaren Säuren.

2. Verfahren für die Herstellung der Verbindung gemäss dem Anspruch 1, dadurch gekennzeichnet, dass man die Verbindung der Formel II

$$Cl-COO-CH_2CH_2-S-(CH_2)_{10}-S-CH_2CH_2-OCO-Cl$$

II

mit dem 3-Pyridylmethanol der Formel III

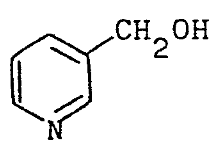

III

reagieren lässt.

3. Verfahren für die Herstellung der Verbindung gemäss dem Anspruch 1, dadurch gekennzeichnet, dass man das 3-Pyridylmethanol-chlorcarbonat der

Formel IV

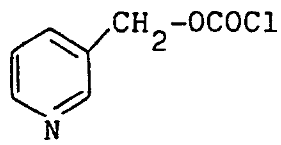

IV

mit dem Bis-hydroxyethylmercapto-1,10-decan der Formel V

$$HO-CH_2CH_2-S-(CH_2)_{10}-S-CH_2CH_2-OH$$

V

umsetzt.

4. Pharmazeutische Zubereitungen mit hypolipidämischen Wirkung, die die Verbindung gemäss dem Anspruch 1 oder dessen Salze von pharmazeutisch annehmbaren Säuren als Wirkstoff enthalten.

Patentansprüche für Österreich

1. Verfahren zur Herstellung eines neues Derivats des Bis-hydroxyethylmercapto-1,10-decans der Formel I

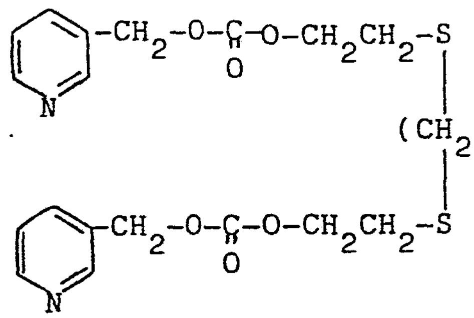

I

sowie dessen Salze von pharmazeutisch annehmbaren Säuren, dadurch gekennzeichnet, dass man a) die Verbindung der Formel II

$$Cl\text{-}COO\text{-}CH_2CH_2\text{-}S\text{-}(CH_2)_{10}\text{-}S\text{-}CH_2CH_2\text{-}OCO\text{-}Cl$$

II

mit dem 3-Pyridylmethanol der Formel III

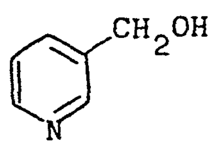

III

reagieren lässt, oder b) das 3-Pyridylmethanolchlorcarbonat (Chlorameisensäure-(3-pyridyl)methylester) der Formel IV

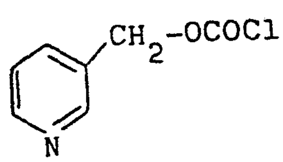

IV

mit dem Bis-hydroxyethylmercapto-1,10-decan der Formel V

$$HO-CH_2CH_2-S-(CH_2)_{10}-S-CH_2CH_2-OH$$

V

umsetzt.

2. Verfahren nach Anspruch1, dadurch gekennzeichnet, dass man die Reaktion zwischen II und III, bwz. zwischen IV und V, in Anwesenheit von Pyridin durchführt.

0060994

Europäisches Patentamt

# EUROPÄISCHER RECHERCHENBERICHT

Nummer der Anmeldung
EP 82 10 1087

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | FR - A - 2 146 138 (L.LAFON)<br>* Patentansprüche; Beispiel 2; Seite 1, Zeilen 32-34 *<br>-- | 1,3,4 |
| A | CHEMICAL ABSTRACTS, Band 89, Nr. 15, 8. Oktober 1978, Zusammenfassung 129411b, Seite 581<br>COLUMBUS, OHIO (US)<br>& ES - A - 456 788 (A.GALLARDO) 1. Februar 1978<br>------------- | 1,3 |

KLASSIFIKATION DER ANMELDUNG (Int Cl.³)

C 07 D 213/30
A 61 K 31/44//
C 07 C 149/20

RECHERCHIERTE SACHGEBIETE (Int Cl.³)

C 07 D 213/00
A 61 K 3/00

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| DEN HAAG | 25. Mai 1982 | NUYTS |